# EUROPEAN PATENT APPLICATION

(11) **EP 2 804 448 A1**
(43) Date of publication of application: **19.11.2014**
(21) Application number: 13736091.3
(22) Date of filing: 11.01.2013
(51) Int. Cl.: H05H 1/24, A61L 2/14, A61L 2/20, B01J 19/08, C02F 1/30

(54) **ACTIVE SPECIES IRRADIATION DEVICE, ACTIVE SPECIES IRRADIATION METHOD AND METHOD FOR FORMING OBJECT HAVING BEEN IRRADIATED WITH ACTIVE SPECIES**

(30) Priority: 13.01.2012 JP 2012005358
(71) Applicant: Osaka University, Suita-shi, Osaka 565-0871 (JP)
(72) Inventor: KITANO Katsuhisa, Suita-shi Osaka 565-0871 (JP); TANI Atsushi, Suita-shi Osaka 565-0871 (JP)
(74) Representative: Viering, Jentschura & Partner Patent- und Rechtsanwälte
(86) International application number: PCT/JP2013/050465
(87) International publication number: WO 2013/105659

(57) **Abstract**

An active species radiation device is for efficiently radiating active species (active oxygen or nitrogen) by using a plasma source not in contact with an irradiated object. An active species radiation device (100) includes: a chamber (110) in which flows of plasma generation gas and active species generation gas enter and which has an active species radiation port (110c) through which active species are radiated; an upstream electrode (120) positioned in a part of the chamber (110) corresponding to an upstream portion of the flow of the plasma generation gas; and a downstream electrode (130) positioned in a part of the chamber (110) corresponding to a more downstream portion of the flow of the plasma generation gas than the upstream electrode (120). The chamber (110) is configured so that the active species generation gas flows into a position between the upstream electrode (120) and the active species radiation port (110c).

## Description

### TECHNICAL FIELD

The present invention relates to an active species radiation device, an active species radiation method, and an active-species-irradiated object producing method.

### BACKGROUND ART

Examples of usage of low-temperature atmospheric pressure plasma include sterilization and disinfection. Because living organisms are present in wet environments, it is important that sterilization be performed in liquids. It has been known that lowering the pH value of liquid and radiating plasma onto the liquid can achieve a strong sterilizing power that reduces the D value (the time it takes to reduce the number of viable bacteria to one tenth) to less than one-hundredth (Patent Document 1), and applying this process to living organisms has been experimented. Further, it is possible to employ an LF plasma jet (Patent Document 2) as a plasma discharge device that functions as a plasma radiation source. Further, a plasma discharge device implements a method (hereinafter, "beforehand blend method") by which oxygen is blended into a discharge-purpose plasma generation gas, prior to the generation of plasma (Patent Document 3).

### [CITATION LIST]

### [Patent Literature]

- [PTL 1]: WO 2009/041049
- [PTL 2]: WO 2008/072390
- [PTL 3]: Japanese Patent Application Laid-Open Publication No. 2002-313599

### SUMMARY OF INVENTION

### [Technical Problem]

When plasma comes in contact with a living organism, however, an unexpected chemical reaction may occur due to free electrons contained in the plasma. Accordingly, bringing plasma into contact with living organisms is not preferable, in view of guaranteeing safety of the living organisms. By using the low-pH method described in Patent Document 1, it is possible to achieve an epochal level of sterilizing power, and the physicochemical mechanism thereof has been revealed. However, the LF plasma jet described in Patent Document 2 is simply used as the plasma discharge device to generate the sterilizing active species, and no particular ingenuity is observed in the plasma discharge device.

In addition, when the plasma discharge device described in Patent Document 3 is used, the purity of the plasma generation gas is degraded in the portion where the electric field is applied, and the degradation makes it more difficult for the electric discharge to occur.

It is an object of the present invention to provide an active species radiation device capable of efficiently radiating active species (active oxygen or active nitrogen) by using a plasma source that is not in contact with irradiated objects, to provide an active species radiation method, and to provide an active-species-irradiated object producing method.

### [Solution to Problem]

To solve the abovementioned problems, an active species radiation device of the present invention includes: a chamber in which flows of plasma generation gas and active species generation gas enter and which has an active species radiation port through which active species are radiated; an upstream electrode that is positioned in a part of the chamber corresponding to an upstream portion of the flow of the plasma generation gas; and a downstream electrode that is positioned in a part of the chamber corresponding to a more downstream portion of the flow of the plasma generation gas than where the upstream electrode is positioned. The chamber is configured so that the active species generation gas flows into a position between the upstream electrode and the active species radiation port.

According to a preferred embodiment of the active species radiation device of the present invention, the chamber includes a plasma generation region where the plasma is generated, and the chamber is configured so that the active species generation gas flows into a position between the inside of the plasma generation region and the active species radiation port.

According to a preferred embodiment of the active species radiation device of the present invention, the chamber is configured so as to have an inflow port for the plasma generation gas and an inflow port for the active species generation gas.

According to a preferred embodiment of the active species radiation device of the present invention, the chamber includes: a first pipe part having an inflow port for the plasma generation gas; a second pipe part having an inflow port for the active species generation gas; and a third pipe part having the active species radiation port, and the chamber is configured so that the first pipe part, the second pipe part, and the third pipe part are joined together in one location so as to form a multi-way tube.

According to a preferred embodiment of the active species radiation device of the present invention, the chamber includes: a fourth pipe part having an inflow port for the plasma generation gas and the active species radiation port; and a fifth pipe part having an inflow port for the active species generation gas and an outflow port for the active species generation gas, and the chamber is configured so that a portion of the fifth pipe part corresponding to the outflow port for the active species generation gas is enclosed in the fourth pipe part.

According to a preferred embodiment of the active species radiation device of the present invention, the chamber is configured so that the active species generation gas flows into a position between the upstream electrode and the downstream electrode.

According to a preferred embodiment of the active species radiation device of the present invention, the chamber is configured so that the active species generation gas flows into a position between the downstream electrode and the active species radiation port.

According to a preferred embodiment of the active species radiation device of the present invention, the active species radiation device further includes a needle member that is connectable to the active species radiation port, and the active species are radiated from a tip end of the needle member.

According to a preferred embodiment of the active species radiation device of the present invention, the active species are of at least one selected from among the following: a hydroxy radical; a superoxide anion radical; a hydroperoxyl radical; singlet oxygen; an oxygen atom; and peroxynitrite (ONOO⁻/ONOOH).

According to a preferred embodiment of the active species radiation device of the present invention, a voltage applied to the upstream electrode is higher than a voltage applied to the downstream electrode.

To solve the abovementioned problems, an active species radiation method of the present invention is for radiating active species by employing an active species radiation device that includes: a chamber in which flows of plasma generation gas and active species generation gas enter and which has an active species radiation port through which active species are radiated; an upstream electrode that is positioned in a part of the chamber corresponding to an upstream portion of the flow of the plasma generation gas; and a downstream electrode that is positioned in a part of the chamber corresponding to a more downstream portion of the flow of the plasma generation gas than where the upstream electrode is positioned. The active species radiation method includes: generating plasma by causing the flow of the plasma generation gas to enter; generating the active species by causing the active species generation gas to flow into a position between the upstream electrode and the active species radiation port; and radiating the active species through the active species radiation port.

According to a preferred embodiment of the active species radiation method of the present invention, as a result of the generating of the plasma, a plasma generation region is formed, and the generating of the active species is realized by causing the active species generation gas to flow into a position between the inside of the plasma generation region and the active species radiation port.

To solve the abovementioned problems, an active-species-irradiated object producing method of the present invention is implemented by employing an active species radiation device that includes: a chamber in which flows of plasma generation gas and active species generation gas enter and which has an active species radiation port through which active species are radiated; an upstream electrode that is positioned in a part of the chamber corresponding to an upstream portion of the flow of the plasma generation gas; and a downstream electrode that is positioned in a part of the chamber corresponding to a more downstream portion of the flow of the plasma generation gas than where the upstream electrode is positioned. The active-species-irradiated object producing method includes: generating plasma by causing the flow of the plasma generation gas to enter; generating the active species by causing the active species generation gas to flow into a position between the upstream electrode and the active species radiation port; and radiating the active species onto a target object through the active species radiation port.

According to a preferred embodiment of the active-species-irradiated object producing method of the present invention, the target object is water, and the active-species-irradiated object is active-species-containing water that contains a larger amount of active species than the water does.

According to a preferred embodiment of the active-species-irradiated object producing method of the present invention, the active species radiation device further includes a needle member that is connectable to the active species radiation port, the radiating of the active species includes radiating the active species onto the inside of the target object from a tip end of the needle member, the target object is a dental root canal, and the active-species-irradiated object is the dental root canal that has been treated with the active species.

### BRIEF DESCRIPTION OF DRAWINGS

[FIG. 1] FIG. 1 is a schematic diagram of an active species radiation device 100 according to the present embodiment.
[FIG. 2] FIG. 2 is a flowchart of an active species radiation method that employs the active species radiation device 100 according to the present embodiment.
[FIG. 3] FIG. 3 is a chart that indicates an active species generation amount by the active species radiation device 100 and an active species generation amount by a conventional device.
[FIG. 4] FIG. 4 is a chart indicating progress in a sterilization process performed by employing the active species radiation device 100.
[FIG. 5] FIG. 5 is a schematic diagram of an active species radiation device 200 according to the present embodiment.
[FIG. 6] FIG. 6 is a schematic diagram of an active species radiation device 300 according to the present embodiment.
[FIG. 7] FIG. 7 is a chart that indicates an active species generation amount by the active species radiation device 300 and an active species generation amount by a conventional device.
[FIG. 8] FIG. 8 is a schematic diagram of an active species radiation device 400 according to the present embodiment.
[FIG. 9] FIG. 9 is a chart indicating a relationship between amounts of oxygen supplied to the active species radiation device 100 and a sterilizing power.
[FIG. 10] FIG. 10 is a chart indicating a comparison between a conventional method (a beforehand blend method) and methods of the present invention (a middle blend method and an afterward blend method).

### DESCRIPTION OF EMBODIMENTS

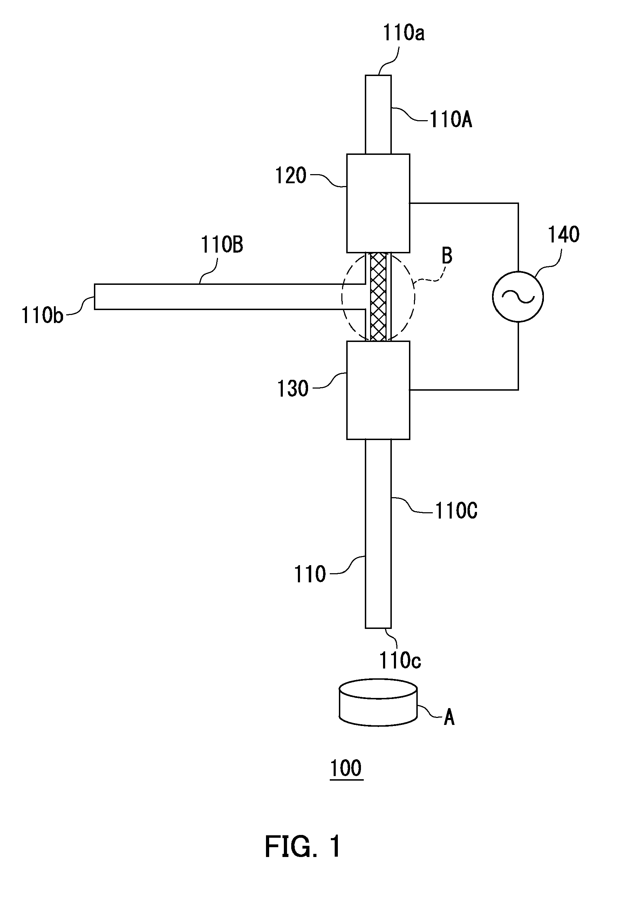

An active species radiation device, an active species radiation method, and an active-species-irradiated object producing method of the present invention will be explained, with reference to FIGS. 1 to 10. It is not intended that the present invention be limited to the configurations described in the embodiments explained below and in the drawings. The present invention also includes any configurations that are equivalent to the described configurations.

### <An active species radiation device 100>

FIG. 1 is a schematic diagram of an active species radiation device 100 according to the present embodiment. By radiating active species onto a target object A with the use of the active species radiation device 100, it is possible to produce an active-species-irradiated object. The active species radiation device 100 includes a chamber 110, an upstream electrode 120, a downstream electrode 130, and a plasma power supply 140.

The chamber 110 is configured so that flows of plasma generation gas and active species generation gas enter therein. The plasma generation gas is helium gas, whereas the active species generation gas is oxygen gas. The chamber 110 has a plasma generation gas inflow port 110a, an active species generation gas inflow port 110b, and an active species radiation port 110c. The active species are radiated through the active species radiation port 110c. The active species are superoxide anion radicals. The active-species-irradiated object is produced by radiating the active species onto the target object A through the active species radiation port 110c. For example, the target object A may be water, whereas the active-species-irradiated object may be active-species-containing water that contains a larger amount of active species than the water does.

The chamber 110 includes a first pipe part 110A, a second pipe part 110B, and a third pipe part 110C. The first pipe part 110A has the plasma generation gas inflow port 110a. The second pipe part 110B has the active species generation gas inflow port 110b. The third pipe part 110C has the active species radiation port 110c. The chamber 110 is configured so that the first pipe part 110A, the second pipe part 110B, and the third pipe part 110C are joined together in one location (a joint portion) so as to form a T-shaped tube. The chamber 110 is a T-shaped glass tube. The outside diameter of the tube of the chamber 110 is 4 mm, whereas the inside diameter thereof is 2 mm. The length of the first pipe part 110A is 10 cm. The length of the second pipe part 110B is 10 cm. The length of the third pipe part 110C is 20 cm.

The first pipe part 110A is positioned in an upstream portion of the flow of the plasma generation gas, whereas the third pipe part 110C is positioned in a downstream portion of the flow of the plasma generation gas. The second pipe part 110B is positioned in an upstream portion of the flow of the active species generation gas, whereas the third pipe part 110C is positioned in a downstream portion of the flow of the active species generation gas. A gas cylinder containing the plasma generation gas is connected, via a gas tube, to the plasma generation gas inflow port 110a of the first pipe part 110A, so that the plasma generation gas gradually flows into the first pipe part 110A from the gas cylinder. A gas cylinder containing the active species generation gas is connected, via a gas tube, to the active species generation gas inflow port 110b of the second pipe part 110B, so that the active species generation gas gradually flows into the second pipe part 110B from the gas cylinder. For example, by controlling the relative ratio between the inflow amount of the plasma generation gas to the chamber 110 and the inflow amount of the active species generation gas to the chamber 110, it is possible to adjust the blend ratio of the active species generation gas.

The upstream electrode 120 is positioned in a part (the first pipe part 110A) of the chamber 110. The upstream electrode 120 is provided coaxially on the outer circumference of the first pipe part 110A. The downstream electrode 130 is positioned in a part (the third pipe part 110C) of the chamber 110. The downstream electrode 130 is provided coaxially on the outer circumference of the third pipe part 110C. The downstream electrode 130 is positioned in a more downstream portion of the flow of the plasma generation gas than where the upstream electrode 120 is positioned. By using a dielectric material (an electrically-insulating material) such as a silica tube or a ceramic tube as a material with which the chamber 110 is configured, it is possible to form an electrode structure in which both the upstream electrode 120 and the downstream 130 are provided with a barrier formed by the dielectric material. By selecting a noble gas such as helium as the plasma generation gas and causing the noble gas to flow into the chamber 110, it is possible to generate stable plasma called atmospheric pressure glow plasma.

The plasma power supply 140 is configured to apply voltages to the upstream electrode 120 and to the downstream electrode 130. The plasma power supply 140 is capable of applying a positive voltage that is in the form of a pulse train and has a predetermined frequency to the upstream electrode 120 and to the downstream electrode 130. The voltage value of the positive voltage in the form of the pulse train that is applied by the plasma power supply 140 may be set at 7 kV for the upstream electrode 120, for example, and so as to have a frequency of approximately 10 kHz, for example. The downstream electrode 130 is arranged to be a ground.

As long as the plasma power supply 140 applies the voltages to the upstream electrode 120 and the downstream electrode 130, the levels of the voltages applied to the upstream electrode 120 and the downstream electrode 130 are not limited. The voltage applied to the upstream electrode 120 may be lower than the voltage applied to the downstream electrode 130. When the voltage applied to the upstream electrode 120 is higher than the voltage applied to the downstream electrode 130, it is possible to generate the plasma in a more stable manner.

Next, the configuration of the chamber 110 will be explained further in detail. When the plasma generation gas is caused to flow in through the plasma generation gas inflow port 110a, and the plasma power supply 140 applies the voltages to the upstream electrode 120 and to the downstream electrode 130, plasma is generated from the plasma generation gas. The chamber includes a region (a plasma generation region B) where the plasma is generated. The chamber is configured so that the active species generation gas flows into a position between the upstream electrode 120 and the downstream electrode 130. More specifically, the chamber 110 is configured so that the active species generation gas flows into the plasma generation region B.

### <An active species radiation method>

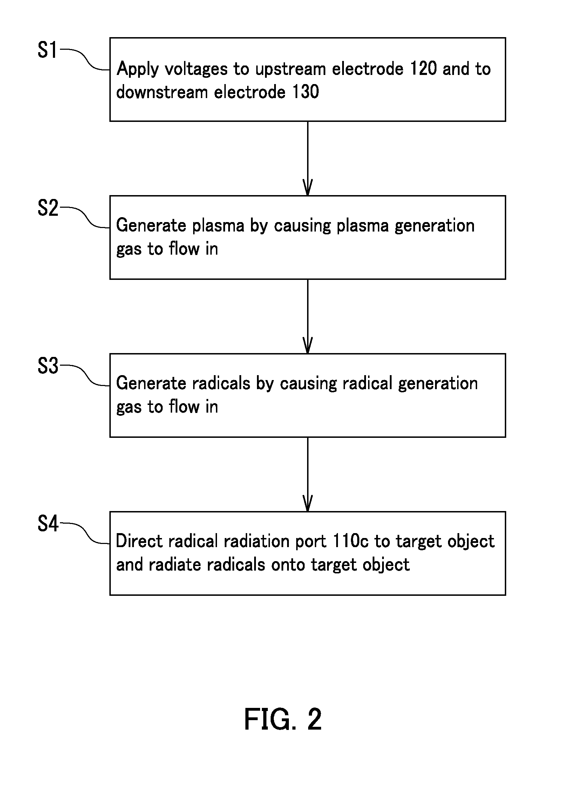

FIG. 2 is a flowchart of an active species radiation method that employs the active species radiation device 100 according to the present embodiment. It is possible to realize an active species radiation method of the present invention by employing the active species radiation device 100 and performing the processes at Steps 1 through 4.

Step 1 (an application step): Voltages are applied to the upstream electrode 120 and to the downstream electrode 130. The voltage value of the positive voltage in the form of a pulse train that is applied by the plasma power supply 140 is set at 7 kV for the upstream electrode 120, for example, and so as to have a frequency of approximately 10 kHz, for example. The downstream electrode 130 is arranged to be a ground.

Step 2 (a plasma generating step): Plasma is generated by causing plasma generation gas to flow in through the plasma generation gas inflow port 110a of the chamber 110. As a result of performing the plasma generating step, the plasma generation region B is formed.

Step 3 (an active species generating step): Active species are generated by causing active species generation gas to flow into a position between the upstream electrode 120 and the downstream electrode 130.

Step 4 (an active species radiating step): The active species are radiated from the active species radiation port 110c. The active species radiation port 110c is directed toward the target object A, so as to radiate the active species onto the target object A.

As a result of performing the processes at Steps 1 through 4, it is possible to produce an active-species-irradiated object from the target object; however, as long as it is possible to generate active species and to radiate the active species onto a target object, the order in which the steps described above are performed is not limited. For example, the plasma generating step may include the application step. It should be noted, however, that it is possible to keep the electric-discharge starting voltage low, by performing Step 3 after performing Step 2 (i.e., after plasma is generated in such a state where only the plasma generation gas is present in the plasma generation region B).

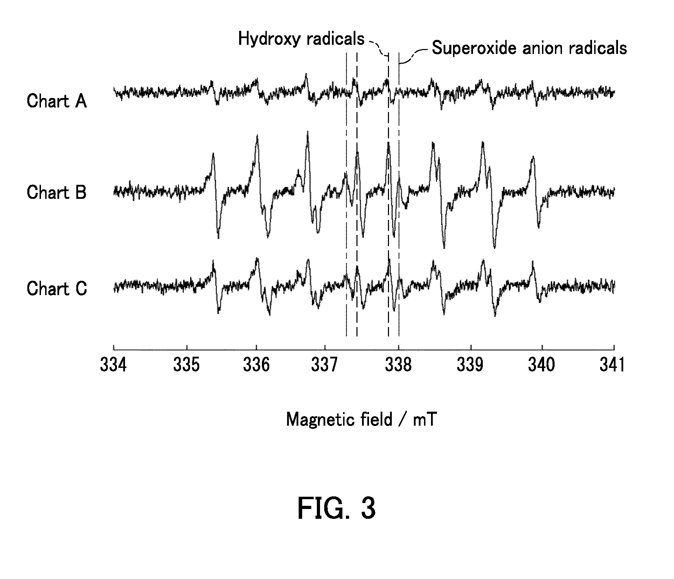

FIG. 3 is a chart that indicates an active species generation amount by the active species radiation device 100 and an active species generation amount by a conventional device. The active species generation amounts were measured by employing an Electron Spin Resonance (ESR) measuring device. In FIG. 3, the horizontal axis expresses a magnetic field (mT).

Chart A indicates the active species generation amount by the conventional device. The conventional device is configured so that active species generation gas is caused to flow into a position on the upstream side of the upstream electrode. Chart B indicates the active species generation amount by the active species radiation device 100. Chart C indicates an active species generation amount by another active species radiation device. The configuration of the other active species radiation device is similar to the configuration of the active species radiation device 100; however, the distance from the plasma generation region B to the active species radiation port 110c in the other active species radiation device is longer than the distance from the plasma generation region B to the active species radiation port 110c in the active species radiation device 100.

As understood from a comparison between Charts A, B, and C, when the active species radiation device 100 was used, at least hydroxy radicals (OH radicals) and superoxide anion radicals (02 anion radicals) were generated. The generation amounts of the hydroxy radicals and the superoxide anion radicals were larger than the generation amounts of these active species observed when the conventional device was used. Further, when the distance from the plasma generation region B to the active species radiation port 110c was shorter, it was possible to introduce the active species to the irradiated object more efficiently than when the distance was longer.

### <The active-species-irradiated object >

By employing the active species radiation device 100 of the present invention and performing the active species radiation method of the present invention, it is possible to apply an active species radiation treatment to the target object A. Thus, from the target object A (e.g., water), it is possible to produce an active-species-irradiated object (e.g., active-species-containing water that contains a larger amount of active species than the water does). Next, details of the active-species-irradiated object (the active-species-treated target object) will be explained.

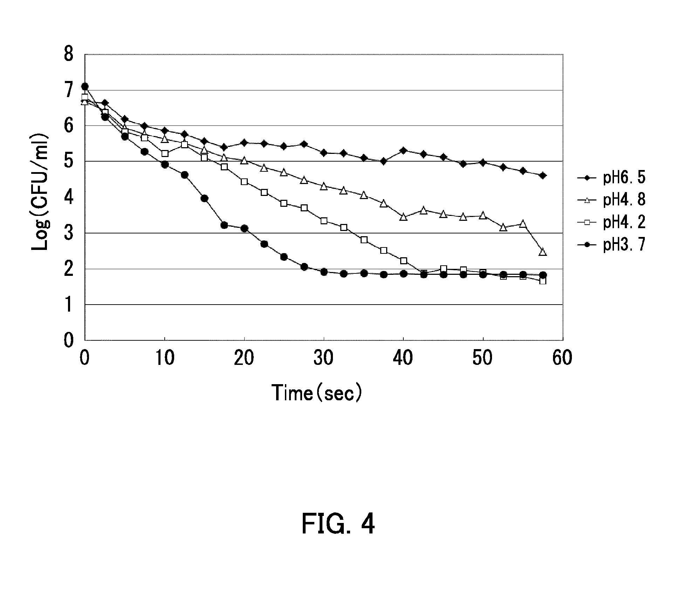

FIG. 4 is a chart indicating progress in a sterilization process performed by employing the active species radiation device 100. Viable bacterial counts of Escherichia coli were evaluated by using a bacterial suspension with Escherichia coli (10⁷ CFU/ml) as the target object A, using helium gas at 450 sccm as the plasma generation gas, and using oxygen gas at 50 sccm as the active species generation gas. When the active species were radiated onto the bacterial suspension with Escherichia coli by employing the active species radiation device 100, the D value (the time it takes to reduce the number of bacteria to one tenth) was 5.6 sec when the pH value of the bacterial suspension was 3.7. The D value was 9.6 sec when the pH value of the bacterial suspension was 4.2. The D value was 15.7 sec when the pH value of the bacterial suspension was 4.8. The D value was 44.8 sec when the pH value of the bacterial suspension was 6.5. It was possible to achieve a strong sterilizing power by lowering the pH value of the bacterial suspension.

The active species radiation device 100 of the present invention has thus been explained, with reference to FIGS. 1 to 4. It is possible to generate a large amount of sterilizing active species by generating a multi-phase flow of the plasma generation gas and the active species generation gas in the intermediate portion of the plasma generation section. Although no contact was made with the liquid surface, it was possible to achieve a sterilizing power that was approximately equivalent to a sterilizing power achieved when an LF plasma jet is brought into contact with liquid surface. Further, it is advantageous in terms of manufacturing of the device because it is possible to achieve a strong sterilizing power even when the distance from the plasma generation region B (the plasma source) to the active species radiation port (the radiation section) is arranged to be approximately 4 m by connecting pipes together.

The active species radiation device 100 is capable of implementing the method (hereinafter, "middle blend method") by which the active species generation gas is caused to flow into a position between the upstream electrode and the downstream electrode. However, as long as the chamber is configured so that active species generation gas is caused to flow into a position between an upstream electrode and an active species radiation port, the structure of the chamber is not limited to a T-shaped structure. For instance, the structure of the chamber may be configured as a coaxial structure.

### <An active species radiation device 200>

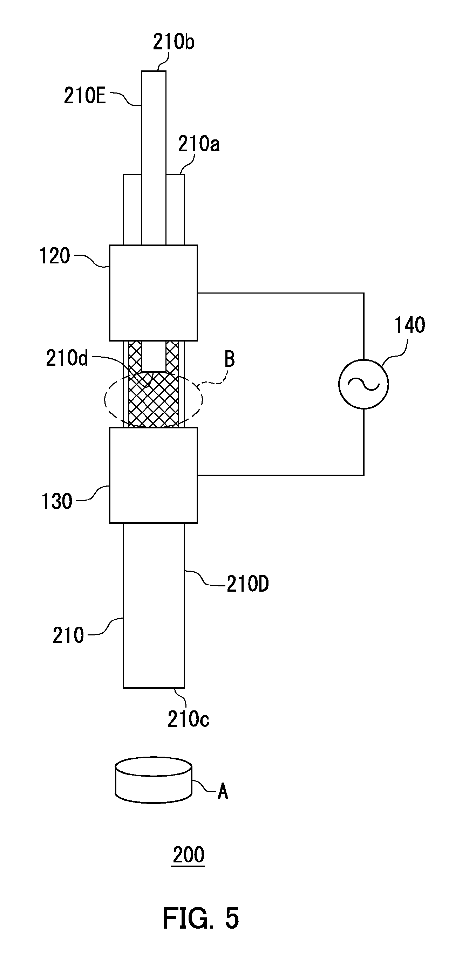

FIG. 5 is a schematic diagram of an active species radiation device 200 according to an embodiment of the present invention. The structure of the chamber included in the active species radiation device 200 is a coaxial structure. By radiating active species onto the target object A from the active species radiation device 200, it is possible to produce an active-species-irradiated object.

The active species radiation device 200 includes a chamber 210, the upstream electrode 120, the downstream electrode 130, and the plasma power supply 140. Because the upstream electrode 120, the downstream electrode 130, and the plasma power supply 140 have the same functions as those of the constituent elements of the active species radiation device 100, detailed explanation thereof will be omitted.

The chamber 210 is configured so that flows of plasma generation gas and active species generation gas enter therein. The plasma generation gas is helium gas, whereas the active species generation gas is oxygen gas. The chamber 210 has a plasma generation gas inflow port 210a, an active species generation gas inflow port 210b, and an active species radiation port 210c. Active species are radiated through the active species radiation port 210c. The active species are superoxide anion radicals. As explained with reference to FIG. 2, by performing the processes at Steps 1 through 4, the active-species-irradiated object is produced by radiating the active species onto the target object A through the active species radiation port 210c. For example, the target object A may be water, whereas the active-species-irradiated object may be active-species-containing water that contains a larger amount of active species than the water does.

The chamber 210 includes a fourth pipe part 210D and a fifth pipe part 210E. The fourth pipe part 210D has the plasma generation gas inflow port 210a and the active species radiation port 210c. The fifth pipe part 210E has the active species generation gas inflow port 210b and an active species generation gas outflow port 210d. The chamber 210 is configured so that a portion of the fifth pipe part 210E corresponding to the active species generation gas outflow port 210d is enclosed in the fourth pipe part 210D. The chamber 210 is a coaxial glass tube. The outside diameter of the tube of the fourth pipe part 210D is 8 mm, whereas the inside diameter thereof is 6 mm. The outside diameter of the tube of the fifth pipe part 210E is 4 mm, whereas the inside diameter thereof is 2 mm. The length of the fourth pipe part 210D is 20 cm. The length of the fifth pipe part 210E is 15 cm.

The chamber 210 is configured so that the active species generation gas flows into a position between the upstream electrode 120 and the downstream electrode 130. Next, a specific positional relationship between the chamber 210, the upstream electrode 120, and the downstream electrode 130 will be explained in detail.

The plasma generation gas inflow port 210a of the fourth pipe part 210D is positioned in an upstream portion of the flow of the plasma generation gas, whereas the active species radiation port 210c of the fourth pipe part 210D is positioned in a downstream portion of the flow of the plasma generation gas. The active species generation gas inflow port 210b of the fifth pipe part 210E is positioned in an upstream portion of the flow of the active species generation gas, whereas the active species generation gas outflow port 210d of the fifth pipe part 210E is positioned in a downstream portion of the flow of the active species generation gas.

A gas cylinder containing the plasma generation gas is connected to the plasma generation gas inflow port 210a of the fourth pipe part 210D, so that the plasma generation gas gradually flows into the fourth pipe part 210D from the gas cylinder. A gas cylinder containing the active species generation gas is connected to the active species generation gas inflow port 210b of the fifth pipe part 210E, so that the active species generation gas gradually flows into the fifth pipe part 210E from the gas cylinder. For example, by controlling the relative ratio between the inflow amount of the plasma generation gas to the chamber 210 and the inflow amount of the active species generation gas to the chamber 210, it is possible to adjust the blend ratio of the active species generation gas.

The upstream electrode 120 is provided in such a portion of the fourth pipe part 210D that corresponds to an upstream portion of the flow of the plasma generation gas. The downstream electrode 130 is provided in such a portion of the fourth pipe part 210D that corresponds to a downstream portion (a more downstream portion than where the upstream electrode 120 is provided) of the flow of the plasma generation gas. The active species generation gas outflow port 210d of the fifth pipe part 210E is provided in a position between the upstream electrode 120 and the downstream electrode 130. With this configuration, the active species radiation device 200 is capable of implementing the method (the middle blend method) by which the active species generation gas is caused to flow into a position between the upstream electrode 120 and the downstream electrode 130.

The active species radiation device 100 and the active species radiation device 200 according to an embodiment of the present invention have thus been explained, with reference to FIGS. 1 to 5. Although the active species radiation device 100 and the active species radiation device 200 implement the "middle blend method", methods are not limited to the "middle blend method", as long as the chamber is configured so that active species generation gas flows into a position between the upstream electrode and the active species radiation port. It is also acceptable to implement a method (hereinafter, "afterward blend method") by which a chamber is configured so that active species generation gas flows into a position between a downstream electrode and an active species radiation port.

### <An active species radiation device 300>

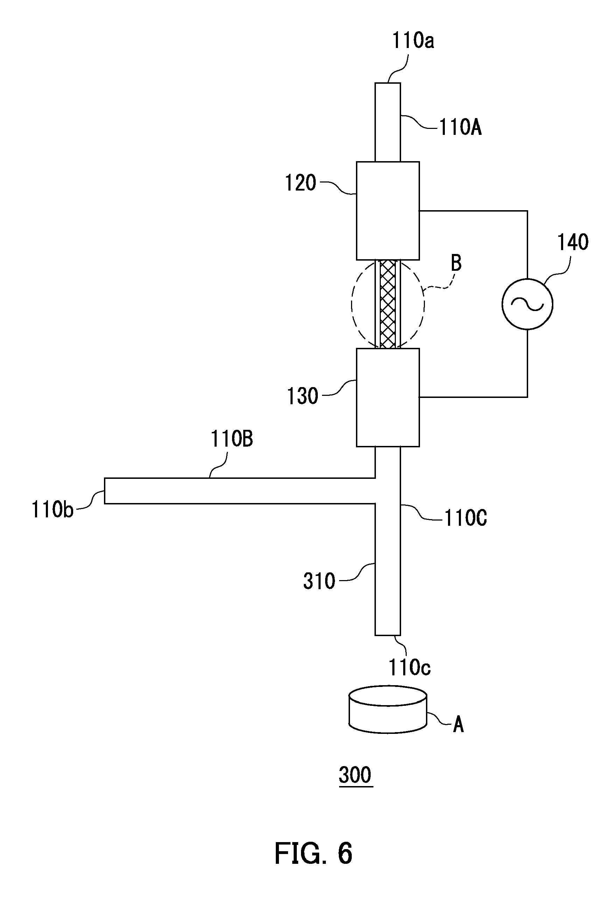

FIG. 6 is a schematic diagram of an active species radiation device 300 according to the present embodiment. By radiating active species onto the target object A with the use of the active species radiation device 300, it is possible to produce an active-species-irradiated object. The active species radiation device 300 includes a chamber 310, the upstream electrode 120, the downstream electrode 130, and the plasma power supply 140. Because the upstream electrode 120, the downstream electrode 130, and the plasma power supply 140 have the same functions as those of the constituent elements of the active species radiation device 100, detailed explanation thereof will be omitted.

The chamber 310 is configured so that flows of plasma generation gas and active species generation gas enter therein. The plasma generation gas is helium gas, whereas the active species generation gas is oxygen gas. The chamber 310 has the plasma generation gas inflow port 110a, the active species generation gas inflow port 110b, and the active species radiation port 110c. Active species are radiated through the active species radiation port 110c. The active species are superoxide anion radicals. As explained with reference to FIG. 2, by performing the processes at Steps 1 through 4, the active-species-irradiated object is produced by radiating the active species onto the target object A through the active species radiation port 110c. For example, the target object A may be water, whereas the active-species-irradiated object may be active-species-containing water that contains a larger amount of active species than the water does.

The chamber 310 includes the first pipe part 110A, the second pipe part 110B, and the third pipe part 110C. The first pipe part 110A has the plasma generation gas inflow port 110a. The second pipe part 110B has the active species generation gas inflow port 110b. The third pipe part 110C has the active species radiation port 110c. The chamber 110 is configured so that the first pipe part 110A, the second pipe part 110B, and the third pipe part 110C are joined together in one location so as to form a T-shaped tube. The chamber 110 is a T-shaped glass tube. The outside diameter of the tube of the chamber 110 is 4 mm, whereas the inside diameter thereof is 2 mm. The length of the first pipe part 110A is 20 cm. The length of the second pipe part 110B is 10 cm. The length of the third pipe part 110C is 10 cm.

The first pipe part 110A is positioned in an upstream portion of the flow of the plasma generation gas, whereas the third pipe part 110C is positioned in a downstream portion of the flow of the plasma generation gas. The second pipe part 110B is positioned in an upstream portion of the flow of the active species generation gas, whereas the third pipe part 110C is positioned in a downstream portion of the flow of the active species generation gas. A gas cylinder containing the plasma generation gas is connected, via a gas tube, to the plasma generation gas inflow port 110a of the first pipe part 110A, so that the plasma generation gas gradually flows into the first pipe part 110A from the gas cylinder. A gas cylinder containing the active species generation gas is connected, via a gas tube, to the active species generation gas inflow port 110b of the second pipe part 110B, so that the active species generation gas gradually flows into the second pipe part 110B from the gas cylinder. For example, by controlling the relative ratio between the inflow amount of the plasma generation gas to the chamber 310 and the inflow amount of the active species generation gas to the chamber 310, it is possible to adjust the blend ratio of the active species generation gas.

The upstream electrode 120 is positioned in a part (the first pipe part 110A) of the chamber 110. The upstream electrode 120 is provided coaxially on the outer circumference of the first pipe part 110A. The downstream electrode 130 is positioned in a part (the first pipe part 110A) of the chamber 110. The downstream electrode 130 is provided coaxially on the outer circumference of the first pipe part 110A. The downstream electrode 130 is positioned in a more downstream portion of the flow of the plasma generation gas than where the upstream electrode 120 is positioned. The first pipe part 110A, the second pipe part 110B, and the third pipe part 110C are joined together in the one location positioned in a more downstream portion than where the downstream electrode 130 is provided.

The plasma power supply 140 is configured to apply voltages to the upstream electrode 120 and the downstream electrode 130. The plasma power supply 140 is capable of applying a positive voltage that is in the form of a pulse train and has a predetermined frequency to the upstream electrode 120 and to the downstream electrode 130. The voltage value of the positive voltage in the form of the pulse train that is applied by the plasma power supply 140 may be set at 7 kV for the upstream electrode 120, for example, and so as to have a frequency of approximately 10 kHz, for example,. The downstream electrode 130 is arranged to be a ground.

As long as the plasma power supply 140 applies the voltages to the upstream electrode 120 and the downstream electrode 130, the levels of the voltages applied to the upstream electrode 120 and the downstream electrode 130 are not limited. The voltage applied to the upstream electrode 120 may be lower than the voltage applied to the downstream electrode 130. When the voltage applied to the upstream electrode 120 is higher than the voltage applied to the downstream electrode 130, it is possible to generate the plasma in a more stable manner.

Next, the configuration of the chamber 310 will be explained further in detail. When the plasma generation gas is caused to flow in through the plasma generation gas inflow port 110a, and the plasma power supply 140 applies the voltages to the upstream electrode 120 and to the downstream electrode 130, plasma is generated from the plasma generation gas. The chamber 310 defines the region (the plasma generation region B) where the plasma is generated. The chamber 310 is configured so that the active species generation gas flows into a position between the downstream electrode 130 and the active species radiation port 110c of the third pipe part 110C. More specifically, the chamber 310 is configured so that the active species generation gas flows into a position between the plasma generation region B and the active species radiation port 110c of the third pipe part 110C.

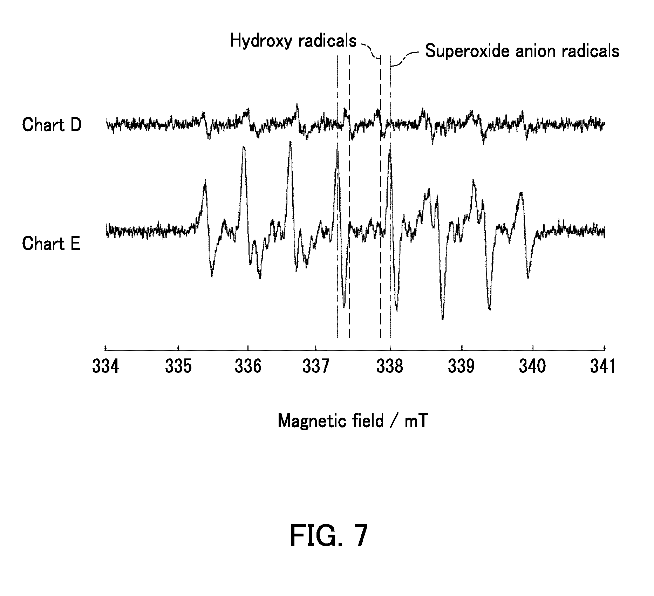

FIG. 7 is a chart that indicates an active species generation amount by the active species radiation device 300 and an active species generation amount by a conventional device. The active species generation amounts were measured by employing an ESR measuring device. In FIG. 7, the horizontal axis expresses a magnetic field (mT).

Chart D indicates the active species generation amount by the conventional device. The conventional device is configured so that active species generation gas is caused to flow into a position on the upstream side of the upstream electrode. Chart E indicates the active species generation amount by the active species radiation device 300.

When the active species radiation device 300 was used, at least hydroxy radicals (OH radicals) and superoxide anion radicals (02 anion radicals) were generated. The generation amount of the superoxide anion radicals was larger than the generation amount of the active species observed when the conventional device was used. Further, in contrast to the example using the active species radiation device 100, when the active species radiation device 300 was used, the generation amount of the superoxide anion radicals was overwhelmingly larger than the generation amount of the hydroxy radicals.

The active species radiation device 300 of the present invention has thus been explained, with reference to FIGS. 6 and 7. By using the active species radiation device 300, it is possible to implement the method (the afterward blend method) by which the chamber is configured so that the active species generation gas flows into a position between the downstream electrode and the active species radiation port. However, as long as the chamber is configured so that the active species generation gas flows into a position between the downstream electrode and the active species radiation port, the structure of the chamber is not limited to a T-shaped structure. For instance, the structure of the chamber may be configured as a coaxial structure.

### <An active species radiation device 400>

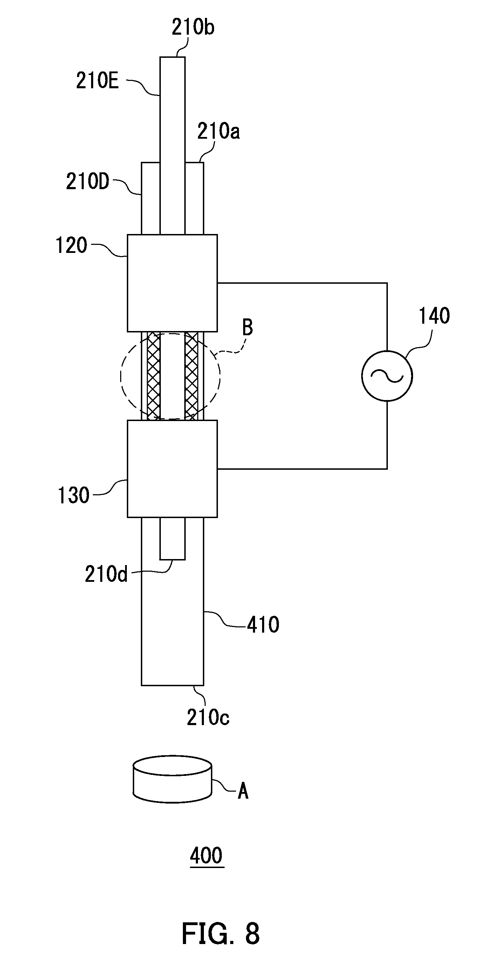

FIG. 8 is a schematic diagram of an active species radiation device 400 according to an embodiment of the present invention. The structure of the chamber included in the active species radiation device 400 is a coaxial structure. By radiating active species onto the target object A from the active species radiation device 400, it is possible to produce an active-species-irradiated object.

The active species radiation device 400 includes a chamber 410, the upstream electrode 120, the downstream electrode 130, and the plasma power supply 140. Because the upstream electrode 120, the downstream electrode 130, and the plasma power supply 140 have the same functions as those of the constituent elements of the active species radiation device 200, detailed explanation thereof will be omitted. Further, because the chamber 410 has the same function as that of the chamber 210 included in the active species radiation device 200, detailed explanation thereof other than the configuration will be omitted.

The chamber 410 is configured so that the active species generation gas flows into a position between the downstream electrode 130 and the active species radiation port 110c. The upstream electrode 120 is provided in such a portion of the fourth pipe part 210D that corresponds to an upstream portion of the flow of the plasma generation gas. The downstream electrode 130 is provided in such a portion of the fourth pipe part 210D that corresponds to a downstream portion (a more downstream portion than where the upstream electrode 120 is provided) of the flow of the plasma generation gas. The active species generation gas outflow port 210d of the fifth pipe part 210E is provided in a position between the downstream electrode 130 and the active species radiation port 110c. With this configuration, the active species radiation device 200 is capable of implementing the method (the afterward blend method) by which the active species generation gas is caused to flow into a position between the downstream electrode 130 and the active species radiation port 110c. As explained with reference to FIG. 2, by performing the processes at Steps 1 through 4, the active-species-irradiated object is produced by radiating the active species onto the target object A through the active species radiation port 110c.

The active species radiation devices, the active species radiation processes, and the active-species-irradiated object producing methods according to the embodiment of the present invention have thus been explained, with reference to FIGS. 1 to 8. It is not intended that the present invention be limited to the configurations described in the embodiments explained above and in the drawings. The present invention also includes any configurations that are equivalent to the described configurations.

In the present embodiment, the plasma generation gas is not limited to helium gas, as long as raw material gas for generating plasma is used. The plasma generation gas may be argon gas, xenon gas, or neon gas. The active species are not limited to superoxide anion radicals. The active species may be active oxygen species or active nitrogen species. For example, the active species may be of at least one selected from among the following: hydroxy radicals (OH radicals); superoxide anion radicals (02 anion radicals); hydroperoxyl radicals; singlet oxygen; oxygen atoms; and peroxynitrite (ONOO-/ONOOH).

The configuration of the active species radiation device is not limited to the T-shaped structure or the coaxial structure, as long as active species generation gas is caused to flow into a position between an upstream electrode and an active species radiation port. Other various configurations are also possible. For example, the chamber included in the active species radiation device may be configured with a three-way tube such as a Y-shaped tube, instead of the T-shaped three-way tube (the T-shaped tube). Further, the chamber may be configured with a multi-way tube structured with four or more pipe parts. Furthermore, the size of the chamber is also arbitrary.

In the present embodiment, the polarity of the voltages applied to the upstream electrode 120 and the downstream electrode 130 is not limited. For example, the voltage applied to the upstream electrode 120 may be higher than the voltage applied to the downstream electrode 130. On the contrary, the voltage applied to the upstream electrode 120 may be lower than the voltage applied to the downstream electrode 130.

In the present embodiment, the shapes of the upstream electrode 120 and the downstream electrode 130 are not limited to those that are coaxial with respect to the pipe parts, as long as it is possible to position the upstream electrode 120 and the downstream electrode 130 each in a part of the chamber. The upstream electrode 120 and the downstream electrode 130 may each have a polygonal shape.

In the present embodiment, the active species generation gas is not limited to oxygen gas, as long as raw material gas for generating active species is used. The active species generation gas may be air. Alternatively, the active species generation gas may be a gas mixture obtained by blending 20% oxygen and 80% nitrogen. Further, the blend ratio of oxygen in the active species generation gas can be adjusted in the range of 1% to 20%. Further, the active species generation gas may be a gas obtained by diluting oxygen gas with helium gas.

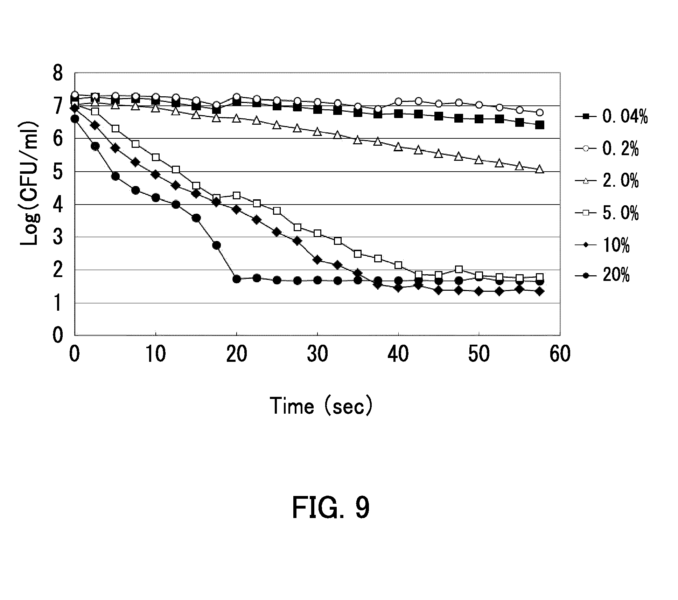

FIG. 9 is a chart indicating a relationship between amounts of oxygen supplied to the active species radiation device 100 and a sterilizing power. A bacterial suspension with Escherichia coli (10⁷ CFU/ml; pH value 3.7) was used as the target object A. Helium gas was used as the plasma generation gas, whereas oxygen gas was used as the active species generation gas. Viable bacterial counts of Escherichia coli were evaluated, while adjusting the flow rates so that the total flow rate of the plasma generation gas and the active species generation gas was equal to 500 sccm, and while varying the oxygen blend ratio from 0.04% to 20%. The D value was 53.5 sec when the oxygen blend ratio was 0.04%. The D value was 51.0 sec when the oxygen blend ratio was 0.2%. The D value was 24.2 sec when the oxygen blend ratio was 2%. The D value was 8.5 sec when the oxygen blend ratio was 5%. The D value was 8.3 sec when the oxygen blend ratio was 10%. It was possible to achieve a strong sterilizing power by raising the oxygen blend ratio.

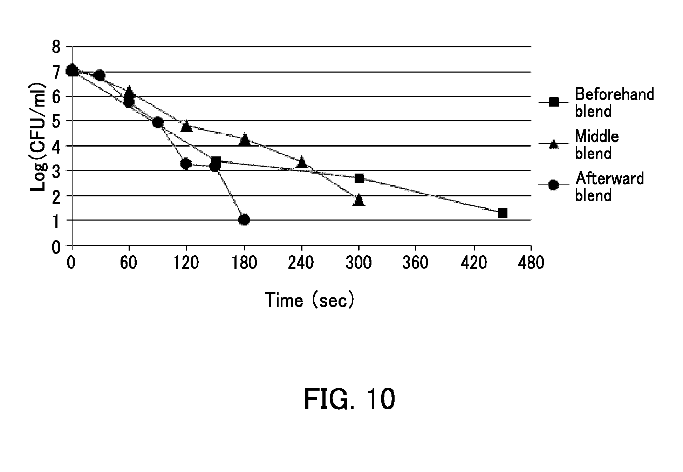

FIG. 10 is a chart indicating a comparison between the conventional method (the beforehand blend method) and the methods according to the present invention (the middle blend method employing the active species radiation device 100 and the afterward blend method employing the active species radiation device 300). A bacterial suspension with Escherichia coli (10⁷ CFU/ml; pH value 3.5) was used as the target object A. Viable bacterial counts of Escherichia coli were evaluated, while adjusting the flow rates so that the total flow rate of the plasma generation gas and the active species generation gas was equal to 2,000 sccm, and while using helium gas at 1,950 sccm as the plasma generation gas, and using oxygen gas at 50 sccm as the active species generation gas.

When the active species were radiated onto the bacterial suspension with Escherichia coli by implementing the conventional method, the D value was 144 sec. In contrast, when the active species were radiated onto the bacterial suspension with Escherichia coli by employing the active species radiation device 100 (i.e., by implementing the middle blend method), the D value was 67 sec. When the active species were radiated onto the bacterial suspension with Escherichia coli by employing the active species radiation device 300 (i.e., by implementing the afterward blend method), the D value was 24 sec. In other words, even if the blend ratios of the active species generation gas were the same, the D value of the middle blend method was approximately 45% of the D value of the beforehand blend method, and a stronger sterilizing power was achieved. Further, even if the blend ratios of the active species generation gas were the same, the D value of the afterward blend method was approximately 15% of the D value of the beforehand blend method, and an even stronger sterilizing power was achieved.

According to the present invention, by introducing the active species generation gas to the position between the upstream electrode and the active species radiation port, it is possible to efficiently generate the active oxygen that is required by the sterilization process employing the plasma source that is not in contact with the target object. In the downstream portion of the chamber, there is a multi-phase flow of the plasma generation gas and the active species generation gas, and a region where the purity of plasma generation gas is high is present. In that region, plasma having high density is efficiently generated due to a short-circuit discharge. When the plasma having high density comes in contact with oxygen gas having high partial pressure, a strong sterilizing power is achieved.

Further, according to the methods of the present invention (the middle blend method and the afterward blend method), it is possible to easily raise the blend ratio of the active species generation gas. In other words, according to the conventional method (the beforehand blend method) by which oxygen is blended with the discharge-purpose plasma generation gas prior to the generation of the plasma, the purity of helium is lowered in the portion where the electric field is applied, which makes it more difficult for an electric discharge to occur. Consequently, according to the conventional method (the beforehand blend method), it is possible to cause an electric discharge with an oxygen blend ratio of only up to approximately several percent.

In contrast, according to the methods of the present invention (the middle blend method and the afterward blend method), because the generated multi-phase flow prevents the active species generation gas having a high discharge voltage from blending with the plasma generation gas having a low discharge voltage (i.e., which causes an electric discharge more easily), an electric discharge occurs relatively more easily. Consequently, as explained above with reference to FIG. 9, it is possible to raise the oxygen blend ratio to 20% or higher when using any of the methods of the present invention. Thus, it is possible to achieve an even stronger sterilizing power, compared to the example where the oxygen blend ratio is only approximately several percent. As explained here, according to the present invention, it is possible to cause a stable electric discharge with the high oxygen blend ratio. It is therefore possible to achieve a sterilizing power that is overwhelmingly stronger than the sterilizing power from the conventional method.

Further, when using any of the active species radiation devices of the present invention, it is possible to adjust the blend radio of the active species generation gas by controlling the relative ratio between the inflow amount of the plasma generation gas to the chamber and the inflow amount of the active species generation gas to the chamber. Consequently, in the situation where a desired level of sterilizing power is not achieved, it is possible to make the sterilizing power stronger, by adjusting the blend ratio of the active species generation gas during the use of the active species radiation device of the present invention.

To cause an electric discharge efficiently with the multi-phase flow, it is desirable to arrange the electrodes in such a manner that the voltages are applied parallel to the flowing direction of the plasma generation gas. Further, when implementing the middle blend method, because the active species generation gas is blended in, the electric discharge occurs relatively less easily on the downstream side of the active species generation gas blend-in portion (i.e., the joint portion where the first pipe part 110A, the second pipe part 110B, and the third pipe part 110C are joined together when the chamber is a three-way tube; the portion in the vicinity of the active species generation gas outflow port 210d when the chamber has a coaxial structure) than on the upstream side thereof. However, an electric discharge occurs more easily in general, when a higher voltage is applied to the downstream electrode, so that the electric field strength on the downstream side is arranged to be higher than that on the upstream side.

When the "middle blend method" of the present invention is used, it is possible to achieve high efficiency in the generation of the active oxygen, because there is a region of sufficient size where the purity of the plasma generation gas is maintained. Also, when the "afterward blend method" is used by which the active species generation gas is blended after the electric discharge is caused by using only the plasma generation gas, there is a region of sufficient size where the purity of the plasma generation gas is maintained. Accordingly, even in the post-discharge gas, because metastable atoms of helium in a high-energy state are less prone to annihilate, and because the metastable atoms come in contact with the active species generation gas, it is possible to achieve high efficiency in the generation of the active oxygen.

Further, under the condition where there is a region of sufficient size where the purity of the plasma generation gas is maintained, it is possible to inhibit ozone formation, because the plasma itself containing high-energy electrons are not in direct contact with the active species generation gas. As a result, it is possible to selectively generate necessary active species (e.g., superoxide anion radicals). By causing the electric discharge only within the glass tube, it is possible to generate the active species in a stable manner and to obtain a strong sterilizing power.

Using the plasma source of a non-contact type has a large advantage in practice, because the plasma generation region does not need to be provided in the vicinity of a lesion and may be provided in a distant position. By using the active species generation source with which the plasma and the liquid are not in contact with each other as described in the present embodiment, it is also possible to produce water containing a large amount of active species.

When any of the active species radiation devices according to the present embodiment is used, the target object may be a dental root canal. Any of the active species radiation devices according to the present embodiment may further include a needle member that is connectable to the active species radiation port. The active species are radiated from the tip end of the needle member. By inserting the tip end of the needle member in the dental root canal, it is possible to efficiently radiate the active species to the inside of the dental root canal. It is therefore possible to produce an active-species-treated dental root canal as an active-species-irradiated object.

When any of the active species radiation devices according to the present embodiment is used, it is also possible to connect a plurality of pipes to the tip end of the needle member. When the plurality of pipes are connected, even in a situation where pressure may rise due to degradation of the conductance of the system inserted in the root canal, it is possible to prevent the pressure in the dental root canal from rising by releasing the gas from other systems to the outside of the dental root canal. It is therefore possible to prevent the onset of emphysema that may be caused by the rise of the gas pressure inside the root canal.

The active species radiation devices, the active species radiation processes, and the active-species-irradiated object producing methods according to the embodiment of the present invention have thus been explained with reference to FIGS. 1 to 10. According to the embodiment of the present invention, by using a tooth, a living organism, and medical equipment as the target object, it is possible to produce an active-species-treated tooth, an active-species-treated living organism, and active-species-treated medical equipment, as active-species-irradiated objects. Due to the germicidal action of the active species, a disinfecting treatment or a germicidal treatment is applied to the tooth, the living organism, and the medical equipment. Thus, it is possible to utilize any of the active species radiation devices and the active species radiation methods according to the present invention for sterilizing purposes.

Further, by using liquid such as water or oil as the target object, it is possible to produce liquid that contains a large amount of active species (liquid that has been treated with the active species radiation) as an active-species-irradiated object. By applying the liquid that has been treated with the active species radiation to a treatment target, a disinfecting treatment or a germicidal treatment is applied to the treatment target. Unlike a method by which active species is directly radiated onto a treatment target, the method by which the liquid that has been treated with the active species radiation is applied to a treatment target is a method by which the active species is indirectly radiated onto the treatment target. However, the disinfecting effect, the sterilizing effect, and the germicidal effect thereof are expected to be equivalent to those of the method by which the active species is directly radiated.

### INDUSTRIAL APPLICABILITY

When any of the active species radiation devices, the active species radiation methods, and the active-species-irradiated object producing methods of the present invention is used, it is possible to efficiently radiate the active species (active oxygen or active nitrogen) by using the plasma source that is not in contact with the irradiated object. Thus, the present invention is suitable for sterilizing the irradiated object or the like.

### REFERENCE SIGNS LIST

- A: target object
- B: plasma generation region
- 100: active species radiation device
- 110: chamber
- 110a: plasma generation gas inflow port
- 110b: active species generation gas inflow port
- 110c: active species radiation port
- 110A: first pipe part
- 110B: second pipe part
- 110C: third pipe part
- 120: upstream electrode
- 130: downstream electrode
- 140: plasma power supply
- 200: active species radiation device
- 210: chamber
- 210a: plasma generation gas inflow port
- 210b: active species generation gas inflow port
- 210c: active species radiation port
- 210D: fourth pipe part
- 210E: fifth pipe part
- 300: active species radiation device
- 310: chamber
- 400: active species radiation device
- 410: chamber

## Claims

1. An active species radiation device comprising:
a chamber in which flows of plasma generation gas and active species generation gas enter and which has an active species radiation port through which active species are radiated;
an upstream electrode that is positioned in a part of the chamber corresponding to an upstream portion of the flow of the plasma generation gas; and
a downstream electrode that is positioned in a part of the chamber corresponding to a more downstream portion of the flow of the plasma generation gas than where the upstream electrode is positioned, wherein
the chamber is configured so that the active species generation gas flows into a position between the upstream electrode and the active species radiation port.

2. The active species radiation device of claim 1, wherein
the chamber includes a plasma generation region where the plasma is generated, and
the chamber is configured so that the active species generation gas flows into a position between an inside of the plasma generation region and the active species radiation port.

3. The active species radiation device of claim 1 or 2, wherein
the chamber is configured so as to have an inflow port for the plasma generation gas and an inflow port for the active species generation gas.

4. The active species radiation device of any one of claims 1 to 3, wherein
the chamber includes:
a first pipe part having an inflow port for the plasma generation gas;
a second pipe part having an inflow port for the active species generation gas; and
a third pipe part having the active species radiation port, and
the chamber is configured so that the first pipe part, the second pipe part, and the third pipe part are joined together in one location so as to form a multi-way tube.

5. The active species radiation device of any one of claims 1 to 3, wherein
the chamber includes:
a fourth pipe part having an inflow port for the plasma generation gas and the active species radiation port; and
a fifth pipe part having an inflow port for the active species generation gas and an outflow port for the active species generation gas, and
the chamber is configured so that a portion of the fifth pipe part corresponding to the outflow port for the active species generation gas is enclosed in the fourth pipe part.

6. The active species radiation device of any one of claims 1 to 5, wherein
the chamber is configured so that the active species generation gas flows into a position between the upstream electrode and the downstream electrode.

7. The active species radiation device of any one of claims 1 to 5, wherein
the chamber is configured so that the active species generation gas flows into a position between the downstream electrode and the active species radiation port.

8. The active species radiation device of any one of claims 1 to 7, further comprising: a needle member that is connectable to the active species radiation port, wherein
the active species are radiated from a tip end of the needle member.

9. The active species radiation device of any one of claims 1 to 8, wherein
the active species are of at least one selected from among the following: a hydroxy radical; a superoxide anion radical; a hydroperoxyl radical; singlet oxygen; an oxygen atom; and peroxynitrite (ONOO⁻/ONOOH).

10. The active species radiation device of any one of claims 1 to 9, wherein
a voltage applied to the upstream electrode is higher than a voltage applied to the downstream electrode.

11. An active species radiation method for radiating active species by employing an active species radiation device that includes:
a chamber in which flows of plasma generation gas and active species generation gas enter and which has an active species radiation port through which active species are radiated;
an upstream electrode that is positioned in a part of the chamber corresponding to an upstream portion of the flow of the plasma generation gas; and
a downstream electrode that is positioned in a part of the chamber corresponding to a more downstream portion of the flow of the plasma generation gas than where the upstream electrode is positioned,
the active species radiation method comprising:
generating plasma by causing the flow of the plasma generation gas to enter;
generating the active species by causing the active species generation gas to flow into a position between the upstream electrode and the active species radiation port; and
radiating the active species through the active species radiation port.

12. The active species radiation method of claim 11, wherein
as a result of the generating of the plasma, a plasma generation region is formed, and
the generating of the active species is realized by causing the active species generation gas to flow into a position between an inside of the plasma generation region and the active species radiation port.

13. An active-species-irradiated object producing method implemented by employing an active species radiation device that includes:
a chamber in which flows of plasma generation gas and active species generation gas enter and which has an active species radiation port through which active species are radiated;
an upstream electrode that is positioned in a part of the chamber corresponding to an upstream portion of the flow of the plasma generation gas; and
a downstream electrode that is positioned in a part of the chamber corresponding to a more downstream portion of the flow of the plasma generation gas than where the upstream electrode is positioned,
the active-species-irradiated object producing method comprising:
generating plasma by causing the flow of the plasma generation gas to enter;
generating the active species by causing the active species generation gas to flow into a position between the upstream electrode and the active species radiation port; and
radiating the active species onto a target object through the active species radiation port.

14. The active-species-irradiated object producing method of claim 13, wherein
the target object is water, and
the active-species-irradiated object is active-species-containing water that contains a larger amount of active species than the water does.

15. The active-species-irradiated object producing method of claim 13, wherein
the active species radiation device further includes a needle member that is connectable to the active species radiation port,
the radiating of the active species includes radiating the active species onto an inside of the target object from a tip end of the needle member,
the target object is a dental root canal, and
the active-species-irradiated object is the dental root canal that has been treated with the active species.
